# EUROPÄISCHE PATENTSCHRIFT

(11) **EP 3 266 427 B1**
(45) Veröffentlichungstag und Bekanntmachung des Hinweises auf die Patenterteilung: **26.08.2020**
(21) Anmeldenummer: 16177791.7
(22) Anmeldetag: 04.07.2016
(51) Int. Cl.: A61F 9/009

(54) **UNTERDRUCKVORRICHTUNG UND VERFAHREN ZUM ÜBERWACHEN EINES OPHTHALMOLOGISCHEN PATIENTENINTERFACES**
VACUUM DEVICE AND METHOD FOR MONITORING AN OPHTHALMOLOGICAL PATIENT INTERFACE
DISPOSITIF A VIDE ET PROCEDE DE SURVEILLANCE D'UNE INTERFACE DE PATIENT OPHTALMOLOGIQUE

(43) Veröffentlichungstag der Anmeldung: 10.01.2018
(73) Patentinhaber: Ziemer Ophthalmic Systems AG, 2562 Port (CH)
(72) Erfinder: RATHJEN, Christian, 28197 Bremen (DE)
(74) Vertreter: Rentsch Partner AG

(56) Entgegenhaltungen:
- EP-A1- 2 913 036
- WO-A1-2016/100439
- US-A1- 2016 106 582

## Beschreibung

### Technisches Gebiet

Die vorliegende Erfindung betrifft eine Unterdruckvorrichtung und ein Verfahren zum Überwachen eines ophthalmologischen Patienteninterfaces. Die vorliegende Erfindung betrifft insbesondere eine Unterdruckvorrichtung zum Fixieren eines ophthalmologischen Patienteninterfaces auf einem Patientenauge und ein Verfahren zum Überwachen des ophthalmologischen Patienteninterfaces.

### Stand der Technik

Zur Behandlung und/oder Diagnose von Augengewebe ist der Einsatz von Strahlungsgeneratoren, insbesondere Lasern, bekannt. Entsprechende Vorrichtungen wie ophthalmologischen Lasereinrichtungen weisen beispielsweise ein Basisgerät mit einer Laser-Lichtquelle zur Erzeugung von Laserpulsen, beispielsweise Femtosekundenlaserpulse, sowie einen Applikationskopf mit einem Projektionsobjektiv auf, der zur Behandlung mit dem Patientenauge gekoppelt wird. Der Applikationskopf kann, beispielsweise über einen Gelenkarm, beweglich mit dem Basisgerät verbunden sein, wobei der Gelenkarm zugleich der optischen Strahlführung von der Laser-Lichtquelle zum Applikationskopf dienen kann. Eine entsprechende Anordnung ist beispielsweise in der EP 1731120 offenbart. Des Weiteren gibt es Geräte, bei denen der Applikationskopf im Basisgerät integriert ist oder bei denen andere Vorrichtungsanordnungen vorgesehen sind.

Die mechanische und optische Kopplung des Applikationskopfes an das Patientenauge, beispielsweise an die Hornhaut und/oder die Sklera des Patientenauges, erfolgt über ein Patienteninterface, wobei das Patienteninterface einen transparenten Kontaktkörper umfassen kann, durch welchen die aus dem Projektionsobjektiv austretenden Laserpulse fokussiert auf oder ins Auge geleitet werden und welcher durch mechanischen Kontakt mit der Hornhaut diese bezüglich des Patienteninterfaces und des Projektionsobjektives fixiert. Alternativ zur Kopplung mittels eines Kontaktkörpers kann eine Flüssigkeitskopplung vorgesehen werden, wobei sich zwischen Hornhaut und Projektionsobjektiv eine Koppelflüssigkeit, beispielsweise physiologische Kochsalzlösung, befindet. Entsprechende Patienteninterfaces sind beispielsweise aus der EP 2030598 bekannt.

Die Kopplung des Patienteninterfaces an das Patientenauge kann mittels Vakuum und einer Unterdruckkavität des Patienteninterfaces erfolgen. Die Unterdruckkavität ist typischerweise ein auf die Hornhaut aufgesetzter Saugring. Die meisten Saugringe haben zwei Dichtlippen. Die Lippen können auf der Sklera, der Sklera und der Hornhaut oder nur auf der Hornhaut angebracht sein. Weiterhin gibt es Varianten, die nur einen Ring besitzen und über dem ganzen Auge ein Vakuum erzeugen, oder Varianten, die aus mehreren Saugkammern/Saugnäpfen bestehen. Der Saugring ist die gebräuchlichste Methode der Befestigung, es gibt jedoch auch andere bekannte Lösungen. In jedem Fall erfolgt die Kopplung an das Patientenauge durch ein Vakuum bzw. ein Unterdruck in mindestens einer Unterdruckkavität des Patienteninterfaces, wobei die Unterdruckkavität entlang ihres Umfangs dichtend auf dem Patientenauge aufliegt und das Patienteninterface so fluidisch dichtend an das Patientenauge koppelt und gegen die Umwelt abdichtet. Die Erzeugung des Unterdrucks kann durch einen Unterdruckgenerator, insbesondere eine Vakuumpumpe bzw. Unterdruckpumpe, erfolgen. Die Kopplung des Patienteninterfaces an den Applikationskopf erfolgt bei bekannten Systemen beispielsweise mittels Schraubverbindung, Bajonettverschlüssen oder Vakuumkupplungen.

Die US 2002/0120285 A1 offenbart eine Klingenführung für ein ophthalmologisches chirurgisches Instrument, welche mittels Vakuum auf dem Patientenauge fixiert ist und den Kontaktdruck zwischen der Sklera und der Klingenführung misst.

Die US 2002/0198553 A1 offenbart ein Patenteninterface und eine Unterdruckvorrichtung mit fluidischer Druckmessung, wobei die Verbindung zum Patienteninterface über eine gemeinsame fluidische Leitung erfolgt.

Die WO 2008/150330 offenbart ein Patienteninterface, welches zur Kopplung mittels Vakuum an das Patientenauge vorgesehen und zweiteilig aufgebaut ist, wobei an einer Koppelstelle zwischen den Teilen Kontaktdrucksensoren angeordnet sind, welche einen Kontaktruck zwischen den Teilen erfassen.

Die 2016/0106582 beschreibt ein System zum Detektieren eines Vakuumverlusts während einer Laseraugenbehandlung, bei welcher die Laserstrahlung durch ein Flüssigkeitsmedium eines flüssigkeitsgefüllten Patienteninterfaces geführt wird. Im System gemäss 2016/0106582 werden mehrere Inputs überwacht, um ein Leck zu detektieren. Die Inputs umfassen einen Videostrom des Auges um nach Luftblasen im Flüssigkeitsmedium zu suchen; Kraftsensoren am Patienteninterface, welche Bewegungen des Patienten detektieren, um diese als frühe Anzeichen von Patientenunruhe und Vorhersage eines möglicherweise bevorstehenden Vakuumlecks zu verwenden; und Vakuumsensoren, die direkt das Ausmass der Saugkraft zwischen dem Patienteninterface und dem Auge messen. Das System nach 2016/0106582 umfasst Steuerelektronik, die die Laserbehandlung anhält oder verzögert, wenn ein Aggregat von allen drei Inputs einem Schwellwert entspricht, der ein signifikantes Vakuumleck anzeigt. Abhängig davon, wie das Aggregat definiert ist, ist es einerseits möglich, dass bei einer vergleichsweise weniger empfindlichen Einstellung ein Abfall von Unterdruck, ohne damit verbundene Blasenbildung, nicht korrekt detektiert wird, oder dass andererseits bei einer vergleichsweise empfindlicheren Einstellung ein kurzzeitiger, temporärer Druckabfall unnötig zu einer Unterbrechung oder einem Abbruch der Laserbehandlung führt.

EP 2913036 beschreibt ein ophthalmologisches Lasersystem mit einem unterdruckbasierten Patienteninterface (Augapfelfixierungseinheit), bei welchem beim Anbringen des Patienteninterfaces auf dem Augapfel der Unterdruck mittels Drucksensoren überwacht wird, um den Kontakt mit dem Augapfel oder einen übergrossen Druck auf den Augapfel zu erkennen. EP 2913036 beschreibt überdies die Detektion der Bewegung von Augenmerkmalen. Wenn sich der Augapfel aufgrund eines Saugunterbruchs bewegt, ist eine Steuereinheit eingerichtet, Laserbestrahlung basierend auf Detektionsergebnissen zu stoppen. Gemäss EP 2913036 wird in einem solchen Fall, verglichen mit der Überwachung des Saugrings mittels Drucksensoren, der Ansaugzustand prompt detektiert.

### Darstellung der Erfindung

Es ist eine Aufgabe der vorliegenden Erfindung eine Unterdruckvorrichtung zum Fixieren eines ophthalmologischen Patienteninterfaces auf einem Patientenauge und ein Verfahren zum Überwachen des ophthalmologischen Patienteninterfaces vorzuschlagen, welche Unterdruckvorrichtung und welches Verfahren, zumindest einige Nachteile der bekannten Systeme nicht aufweisen. Es ist insbesondere eine Aufgabe der vorliegenden Erfindung eine Unterdruckvorrichtung und ein Verfahren zum Überwachen des ophthalmologischen Patienteninterfaces vorzuschlagen, welche unnötige Unterbrechungen von Laserbehandlungen möglichst vermeiden.

Gemäss der vorliegenden Erfindung werden diese Ziele durch die Merkmale der unabhängigen Ansprüche erreicht. Weitere vorteilhafte Ausführungsformen gehen ausserdem aus den abhängigen Ansprüchen und der Beschreibung hervor.

Eine Unterdruckvorrichtung zum Fixieren eines ophthalmologischen Patienteninterfaces auf einem Patientenauge umfasst einen Unterdruckgenerator und eine Unterdruckschnittstelle zur fluidischen Kopplung des Unterdruckgenerators an eine Unterdruckkavität des Patienteninterfaces.

Die oben genannten Ziele werden durch die vorliegende Erfindung insbesondere dadurch erreicht, dass die Unterdruckvorrichtung einen an das Patienteninterface fluidisch ankoppelbaren internen Drucksensor und/oder eine Druckmessschnittstelle zur signaltechnischen Ankopplung eines externen Drucksensors des Patienteninterfaces umfasst, und zudem einen Bewegungsdetektor aufweist, der eingerichtet ist, Bewegungen des Patientenauges zu detektieren. Die Unterdruckvorrichtung umfasst überdies eine Steuereinheit, die eingerichtet ist, aufgrund des vom angekoppelten Drucksensor ermittelten Drucks eine fehlerhafte fluidische Kopplung der Unterdruckkavität zu detektieren, und ein Steuersignal zum Abbrechen einer durch eine ophthalmologische Behandlungsvorrichtung durchgeführten ophthalmogischen Behandlung zu erzeugen, wenn gleichzeitig mit der detektierten fehlerhaften fluidischen Kopplung der Unterdruckkavität vom Bewegungsdetektor eine Augenbewegung detektiert wird.

In einer Ausführungsvariante ist die Steuereinheit eingerichtet, ohne Unterbruch der ophthalmogischen Behandlung ein Warnsignal zu erzeugen, wenn während einer detektierten fehlerhaften fluidischen Kopplung der Unterdruckkavität keine Augenbewegung detektiert wird.

In einer Ausführungsvariante umfasst der Bewegungsdetektor einen Videosensor und eine mit dem Videosensor verbundene Verarbeitungseinheit, welche eingerichtet ist, die Augenbewegungen aufgrund von vom Videosensor gelieferten Videosignalen zu detektieren.

In einer Ausführungsvariante ist der Bewegungsdetektor eingerichtet, Bewegungen des Patientenauges zu detektieren, welche das Patientenauge relativ zu einer starren Beobachtungsachse bewegen.

In einer Ausführungsvariante ist die Steuereinheit eingerichtet, das Steuersignal zum Abbrechen der ophthalmogischen Behandlung zu erzeugen, wenn vom Bewegungsdetektor eine Augenbewegung detektiert wird, die über einem definierten Toleranzschwellwert liegt.

In einer Ausführungsvariante umfasst die Unterdruckvorrichtung eine mit dem internen Drucksensor fluidisch verbundene Drucksensorschnittstelle, die eingerichtet ist, den internen Drucksensor separat zu der Unterdruckschnittstelle fluidisch mit dem Patienteninterface zu koppeln.

In einer Ausführungsvariante ist die Druckmessschnittstelle eingerichtet zur signaltechnischen Ankopplung eines am Patienteninterface angeordneten externen Kontaktdrucksensors.

In einer Ausführungsvariante ist die Steuereinheit eingerichtet, die fehlerhafte fluidische Kopplung der Unterdruckkavität durch Detektion einer Abweichung zwischen dem mittels des angekoppelten Drucksensors ermittelten Drucks und einem Referenzdruck, und/oder durch Detektion eines Abfalls des Drucks in Funktion der Zeit, zu detektieren.

In einer Ausführungsvariante umfasst die Unterdruckvorrichtung einen mit der Unterdruckschnittstelle fluidisch gekoppelten und mit der Steuereinheit verbundenen zweiten Drucksensor, und die Steuereinheit ist eigerichtet, die fehlerhafte fluidische Kopplung der Unterdruckkavität durch Vergleichen des ermittelten Drucks mit einem vom zweiten Drucksensor ermittelten zweiten Druck zu detektieren.

In einer Ausführungsvariante umfasst der Bewegungsdetektor eine Sensorvorrichtung, welche eingerichtet ist, die Augenbewegungen aufgrund von Distanzveränderungen des Patientenauges relativ zum Patienteninterface zu detektieren.

In einer Ausführungsvariante ist die Steuereinheit eingerichtet, aufgrund einer detektierten Augenbewegung ein Korrektursignal zur Repositionierung und Fortführung der ophthalmogischen Behandlung zu erzeugen, wenn eine Beendigung der Augenbewegung und keine fehlerhafte fluidische Kopplung der Unterdruckkavität detektiert wird.

Neben der Unterdruckvorrichtung zum Fixieren eines ophthalmologischen Patienteninterfaces auf einem Patientenauge betrifft die vorliegende Anmeldung auch ein Verfahren zum Überwachen des ophthalmologischen Patienteninterfaces, das mittels eines, durch den Unterdruckgenerator in einer Unterdruckkavität des Patienteninterfaces hergestellten, Unterdrucks auf dem Patientenauge fixiert ist. Das Verfahren umfasst: Ermitteln eines Drucks in der Unterdruckkavität mittels eines fluidisch an das Patienteninterface angekoppelten Drucksensors und/oder eines Kontaktdrucksensors des Patienteninterfaces; Detektieren von Bewegungen des Patientenauges mittels eines Bewegungsdetektors; Detektieren durch eine Steuereinheit einer fehlerhaften fluidischen Kopplung der Unterdruckkavität an den Unterdruckgenerator aufgrund des ermittelten Drucks; und Erzeugen durch die Steuereinheit eines Steuersignals zum Abbrechen einer durch eine ophthalmologische Behandlungsvorrichtung durchgeführten ophthalmogischen Behandlung, wenn gleichzeitig mit der detektierten fehlerhaften fluidischen Kopplung der Unterdruckkavität vom Bewegungsdetektor eine Augenbewegung detektiert wird.

In einer Ausführungsvariante erzeugt die Steuereinheit, ohne Unterbruch der ophthalmogischen Behandlung, ein Warnsignal, wenn während einer detektierten fehlerhaften fluidischen Kopplung der Unterdruckkavität keine Augenbewegung detektiert wird.

In einer Ausführungsvariante erfolgt eine Videoerfassung des Patientenauges durch einen Videosensor, und die Augenbewegungen werden durch die Steuereinheit aufgrund der Videoerfassung detektiert.

In einer Ausführungsvariante erzeugt die Steuereinheit das Steuersignal zum Abbrechen der ophthalmogischen Behandlung, wenn vom Bewegungsdetektor eine Augenbewegung detektiert wird, die über einem definierten Toleranzschwellwert liegt.

Die vorliegende Anmeldung betrifft auch eine ophthalmologische Behandlungsvorrichtung, die ein Lasersystem zur ophthalmologischen Behandlung eines Patientenauges, einen Applikationskopf, und ein Patienteninterface zum Anbringen des Applikationskopfes auf dem Patientenauge umfasst. Die ophthalmologische Behandlungsvorrichtung umfasst überdies die obenstehend angeführte Unterdruckvorrichtung zum Fixieren des ophthalmologischen Patienteninterfaces auf dem Patientenauge.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
- Figur 1:: zeigt ein Blockdiagramm einer ophthalmologischen Anordnung mit einer Unterdruckvorrichtung und einem damit fluidisch gekoppelten Patienteninterface.
- Figur 2:: zeigt schematisch im Querschnitt eine Ausführungsform eines an einem Patientenauge fixierten Patienteninterfaces, das fluidisch mit einer Unterdruckvorrichtung gekoppelt ist.
- Figur 3:: zeigt ein Blockdiagramm einer Unterdruckvorrichtung mit einem Unterdruckgenerator und einer Unterdruckschnittstelle zur fluidischen Kopplung des Unterdruckgenerators an eine Unterdruckkavität eines Patienteninterfaces.
- Figur 4:: zeigt ein Blockdiagramm einer weiteren ophthalmologischen Anordnung mit einer Unterdruckvorrichtung und einem damit fluidisch gekoppelten Patienteninterface.
- Figur 5:: zeigt verschiedene Druckverläufe in Funktion der Zeit.
- Figur 6:: zeigt ein Flussdiagramm, das Prozesse und Schritte zum Überwachen eines ophthalmologischen Patienteninterfaces illustriert.

### Wege zur Ausführung der Erfindung

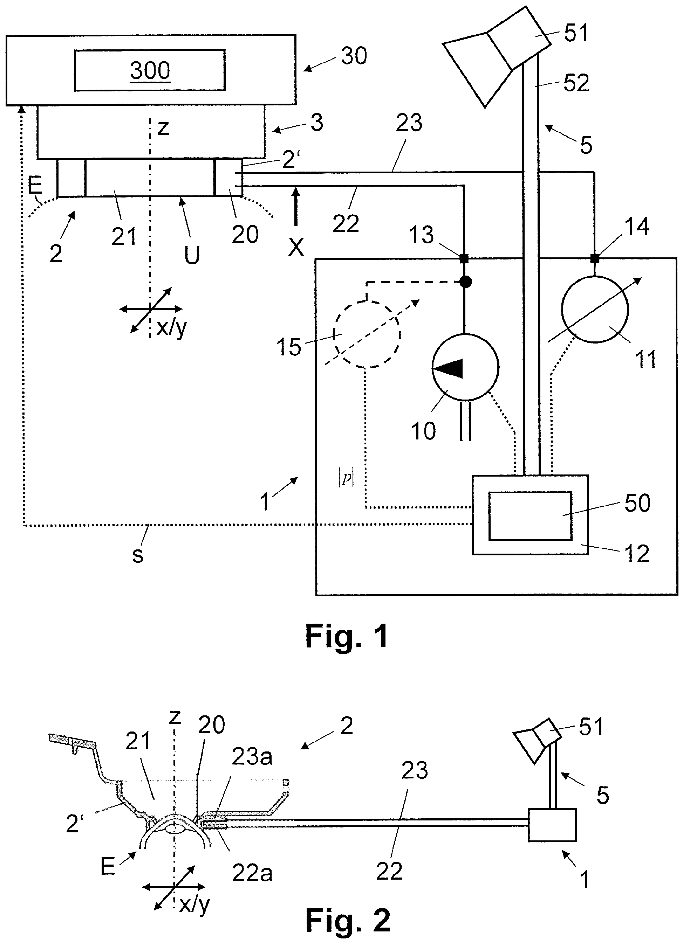

In der Figur 1 bezeichnet das Bezugszeichen 1 eine Unterdruckvorrichtung und das Bezugszeichen 2 ein an die Unterdruckvorrichtung 1 angeschlossenes Patienteninterface in schematischem Querschnitt. Zur Durchführung einer ophthalmogischen Behandlung ist das Patienteninterface 2 mit einem ophthalmologischen Applikationskopf 3 einer ophthalmologischen Behandlungsvorrichtung 30 gekoppelt. Die Unterdruckvorrichtung 1 und das Patienteninterface 2 bilden gemeinsam eine ophthalmologische Anordnung. Die ophthalmologische Behandlungsvorrichtung 30 umfasst ein Lasersystem 300, das eingerichtet ist, zur ophthalmogischen Behandlung des Patientenauges E Laserpulse zu erzeugen und über den Applikationskopf 3 und das Patienteninterface 2 fokussiert auf und in das Patientenauge E zu strahlen.

Im Anwendungszustand liegt das Patienteninterface 2 mit seiner Unterseite U des Patienteninterfacekörpers 2' auf der Hornhaut des Patientenauges E auf. Der Patienteninterfacekörper 2' des Patienteninterfaces 2 weist einen zum Beispiel zylindrischen Innenraum 21 auf, welcher sich im Anwendungszustand zwischen der Hornhautoberfläche des Patientenauges E und dem Applikationskopf 3 befindet und beispielsweise mit physiologischer Kochsalzlösung als Koppelflüssigkeit und optisches Übertragungsmedium gefüllt sein kann. Konzentrisch um den Innenraum 21 ist ein Saugring angeordnet, welcher im Anwendungszustand ebenfalls auf der Hornhaut des Patientenauges E aufliegt und dessen Innenraum mindestens eine Unterdruckkavität 20 bildet, die beispielsweise ringförmige ausgestaltet ist. Zur Kopplung des Patienteninterfaces 2 bzw. des Patienteninterfacekörpers 2' an das Patientenauge E wird in der Unterdruckkavität 20 ein Unterdruck bzw. Vakuum erzeugt, welches so das Patienteninterface 2 auf dem Patientenauge E fixiert.

Die Unterdruckvorrichtung 1 umfasst einen Unterdruckgenerator 10, der typischerweise durch eine Vakuumpumpe gebildet wird, sowie eine Unterdruckschnittstelle 13, die fluidisch mit dem Unterdruckgenerator 10 gekoppelt ist. Der Unterdruckgenerator 10 und die Unterdruckkavität 20 des Patienteninterfaces 2 sind über die Unterdruckverbindungsleitung 22 fluidisch miteinander gekoppelt. Auf der Seite des Patienteninterfaces 2 ist die Unterdruckverbindungsleitung 22 mit einem Ende entfernbar oder fest fluidisch mit der Unterdruckkavität 20 des Patienteninterfaces 2 gekoppelt. Auf der Seite der Unterdruckvorrichtung 1 ist die Unterdruckverbindungsleitung 22 mit ihrem anderen Ende über die Unterdruckschnittstelle 13 fluidisch mit dem Unterdruckgenerator 10 gekoppelt.

Die Unterdruckvorrichtung 1 umfasst zudem einen fluidischen Drucksensor 11 und eine Drucksensorschnittstelle 14, die fluidisch mit dem Drucksensor 11 gekoppelt ist. Der Drucksensor 11 und die Unterdruckkavität 20 des Patienteninterfaces 2 sind über die Drucksensorverbindungsleitung 23 fluidisch miteinander gekoppelt, separat von der Unterdruckverbindungsleitung 22. Auf der Seite des Patienteninterfaces 2 ist die Drucksensorverbindungsleitung 23 mit einem Ende entfernbar oder fest fluidisch mit der Unterdruckkavität 20 des Patienteninterfaces 2 gekoppelt. Auf der Seite der Unterdruckvorrichtung 1 ist die Drucksensorverbindungsleitung 23 mit ihrem anderen Ende über die Drucksensorschnittstelle 14 fluidisch mit dem Drucksensor 11 gekoppelt.

Die Unterdruckverbindungsleitung 22 und die Drucksensorverbindungsleitung 23 sind mit einem Ende jeweils separat fluidisch an die Unterdruckkavität 20 gekoppelt, wobei sich die fluidisch separate Kopplung jeweils auf den gesamten fluidischen Pfad und insbesondere bis zur Unterdruckkavität 20 erstreckt.

Die Unterdruckschnittstelle 13 und Drucksensorschnittstelle 14 sind beispielsweise als lösbare fluidische Steckverbinder oder Koppler, z. B. fluidische Kupplungsbuchsen, ausgebildet. Die Unterdruckverbindungsleitung 22 und die Drucksensorverbindungsleitung 23 weisen jeweils entsprechende fluidische Koppelelemente auf, z. B. fluidische Steckverbinder, die zur lösbaren Kopplung mit der vorrichtungsseitigen Unterdruckschnittstelle 13 bzw. der vorrichtungsseitigen Drucksensorschnittstelle 14 vorgesehen sind.

Die Unterdruckvorrichtung 1 umfasst überdies eine Steuereinheit 12, welche eine Verarbeitungseinheit 50 zur Steuerung der Funktion der Unterdruckvorrichtung 2 umfasst. Die Verarbeitungseinheit 50 ist als elektronische Schaltung ausgebildet und umfasst eine Logikschaltung, z.B. ein ASIC (Application Specific Integrated Circuit) oder ein FPGA (Field Programmable Gate Array), und/oder einen oder mehrere Mikroprozessoren mit gespeichertem Programmcode zur Steuerung der Mikroprozessoren derart, dass diese die nachfolgend beschriebenen Funktionen der Steuereinheit 12 und des nachfolgend angeführten Bewegungsdetektors 5 ausführen. Wie in den Figuren 1 bis 4 schematisch dargestellt ist, ist die Unterdruckvorrichtung 1 mit einem Bewegungsdetektor 5 versehen, der eingerichtet ist Bewegungen des Patientenauges E zu detektieren. Der Bewegungsdetektor 5 ist eingerichtet, Bewegungen des Patientenauges E relativ zu einer festen Beobachtungsachse, beispielsweise die optische Achse z eines Projektionsobjektivs der ophthalmologischen Behandlungsvorrichtung 30, zu detektieren, sowohl in Bewegungsrichtungen (x, y), die in einer zur Beobachtungs- respektive optischen Achse z normalen x/y-Ebene verlaufen und einer Verschiebung des Patientenauges E (respektive einem entsprechenden Verrutschen des Patienteninterfaces 2) zur Beobachtungs- respektive optischen Achse z entsprechen, als auch in Bewegungsrichtungen (z), die entlang der Beobachtungsrespektive optischen Achse z verlaufen und einer Distanzveränderung zum Patienteninterface 2 respektive zum Applikationskopf 3 (oder einem Abheben des Patienteninterfaces 2) entsprechen. Abhängig von der Ausführungsvariante umfasst der Bewegungsdetektor 5 eine oder mehrere Sensorvorrichtungen 51 für bildgebende oder strahlbasierte Detektionsverfahren, z.B. einen Videosensor zur Videoerfassung des Patientenauges E und/oder Lichtsender und Lichtdetektoren für strahlbasierte OCT (Optical Coherence Tomography) Systeme oder Triangulationssysteme, etc. Der Videosensor ist beispielsweise als CCD Kamera ausgeführt (Charge Coupled Device) und liefert Videosignale vom überwachten Patientenauge E. Abhängig von Ausführungsvariante und/oder Konfiguration ist die Sensorvorrichtung 51 an der Unterdruckvorrichtung 1 angebracht, beispielsweise mittels eines Trägers, oder, fest oder entfernbar an der ophthalmologischen Behandlungsvorrichtung 30, beispielsweise am Applikationskopf 3, angebracht oder integriert. Die Messsignale, z.B. Videosignale, werden von der Sensorvorrichtung 51 laufend über eine Messsignalleitung 52, z.B. eine Videosignalleitung, an die Verarbeitungseinheit 50 übertragen, wo sie zur Detektion von Augenbewegungen ausgewertet werden. Zur Detektion von Augenbewegungen aufgrund von Videosignalen ermittelt die Verarbeitungseinheit 50 in den durch die Videosignale definierten Videoframes des Patientenauges E laufend örtliche Veränderungen von Referenzmerkmalen des betreffenden Patientenauges E, z.B. Pupille, Iris und/oder charakteristische Detailmerkmale der Iris, bezüglich vorgängig erfassten und gespeicherten Referenzpositionen. Zur Detektion von Augenbewegungen mittels OCT werden sich wiederholende räumliche Scanns des Patientenauges E durchgeführt, z.B. Kreisschnitte durch die Cornea oder ein anderer definierter Volumenscann eines definierten Augenbereichs, und durch die Verarbeitungseinheit 50 laufend auf inhaltliche Abweichungen (an den gleichen Stellen) untersucht, die eine Augenbewegung anzeigen. Dadurch erkennt und detektiert die Verarbeitungseinheit 50 Bewegungen des Patientenauges E relativ zur festen Beobachtungs- respektive optischen Achse z. Durch die strahlbasieren OCT respektive Triangulationsverfahren werden zusätzlich zu lateralen Verschiebungen in x/y-Richtung auch Augenbewegungen durch Distanzveränderungen des Patientenauges E zum Patienteninterface 2 respektive zum Applikationskopf 3 in z-Richtung detektiert. Der Fachmann wird verstehen, dass weitere Bewegungsdetektoren 5 zur Detektion von Bewegungen des Patientenauges E relativ zum Patienteninterface in seitlicher x/y-Richtung oder mit Distanzveränderung in z-Richtung eingesetzt werden können. In einer Ausführungsvariante, zeigt der Bewegungsdetektor 5 respektive die Verarbeitungseinheit 50 eine detektierte Augenbewegung an, wenn Positionsänderungen von Augenstrukturen festgestellt werden, die über einem definierten Toleranzschwellwert liegen, beispielsweise über einer definierten Distanzgrenze d, z.B. d> 1mm, bezüglich der Beobachtungsachse z in x, y oder z-Richtung.

Figur 2 stellt schematisch einen Teil eines Patienteninterfaces 2 gemeinsam mit einem Patientenauge E dar. Das Patienteninterface 2 besitzt einen Patienteninterfacekörper 2' mit einer ringförmigen Unterdruckkavität 20 und einem Innenraum 21. In die Unterdruckkavität 20 münden separat zwei fluidische Anschlussstutzen 22a, 23a, welche mit den patienteninterfaceseitigen Ende der Unterdruckanschlussleitung 22 und der Drucksensorverbindungsleitung 23 in grundsätzlich bekannter Weise fluidisch dicht verbunden sind, z. B. durch Kleben, Ultraschall-Schweissen, oder durch Reibschluss. Die anderen Enden der fluidischen Unterdruckanschlussleitung 22 und Drucksensorverbindungsleitung 23 sind mit der Unterdruckvorrichtung 1 gekoppelt, beispielsweise mittels Steckverbindern, die im Betrieb mit einem entsprechenden Patienteninterfacekoppler der Unterdruckvorrichtung 1 in Form einer fluidischen Kupplungsbuchse gekoppelt sind.

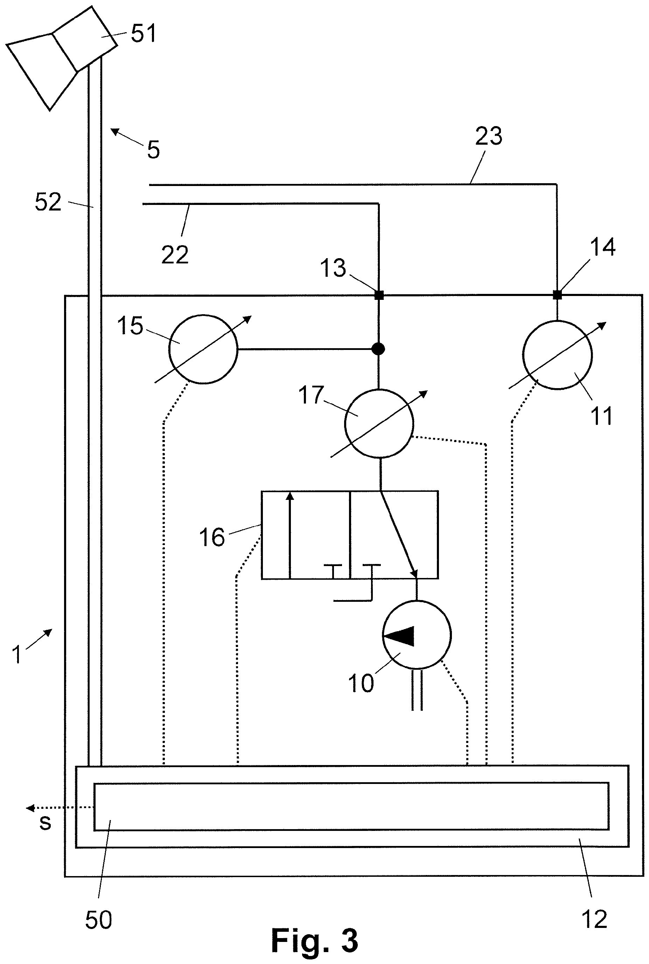

Figur 3 zeigt ein Ausführungsbeispiel der Unterdruckvorrichtung 1, die in grundsätzlich ähnlicher Weise aufgebaut ist wie die Unterdruckvorrichtung 1 gemäss Figur 1, jedoch zusätzlich eine operativ mit der Steuereinheit gekoppelte und durch die Steuereinheit 12 angesteuerte Ventileinheit 16 umfasst.

Über die Ventileinheit 16 wird die Unterdruckversorgungsleitung 22 alternativ mit dem Unterdruckgenerator 10 (dargestellte Stellung) zu Aufbau und Aufrechterhaltung des Unterdrucks oder mit der Umgebung zur Entlüftung und zum Abbau des Unterdrucks in der Unterdruckkavität 20 verbunden.

Die in Figur 3 dargestellte Ausführungsform der Unterdruckvorrichtung 11 umfasst ferner einen optionalen Durchflusssensor 17, welcher fluidisch zwischen der Unterdruckschnittstelle 13 und der Ventileinheit 16 angeordnet ist. Der Durchflusssensor 17 dient der Detektion von Fehlerzuständen, insbesondere von temporären oder dauerhaften Leckagen, welche beim Absaugen von Luft aus der Unterdruckkavität 20 einen erhöhten Luftfluss bewirken. Bei einem Pseudovakuum, bei dem ein ausreichender Unterdruck bzw. ein Vakuum in der Unterdruckkavität 20 nur scheinbar, tatsächlich aber nicht vorhanden ist, zum Beispiel aufgrund eines Abknickens der Unterdruckverbindungsleitung 22 während des Ansaugvorgangs an einer in Figur 1 beispielhaft mit "X" markierten Stelle oder verursacht durch Bindegewebe oder Sterilabdeckungen, misst der zweite Drucksensor 15 bei Betrieb des Unterdruckgenerators 10 einen (normalen) Unterdruck, dabei erfolgt jedoch im Vergleich zur Situation ohne Pseudovakuum kein oder nur ein reduzierter Luftdurchfluss durch den Durchflusssensor 17, was eine Detektion des Pseudovakuums ermöglicht.

Die in Figur 1 und Figur 3 dargestellten Ausführungsformen einer Unterdruckvorrichtung 1 können in verschiedener Weise modifiziert werden. So kann der Drucksensor 11 anstelle innerhalb eines Gehäuses (nicht referenziert) der Steuereinheit 1 unmittelbar am bzw. im Patienteninterface 2 angeordnet sein, wobei die Drucksensorverbindungsleitung 23 entfällt und stattdessen eine elektrische Drucksensor-Anschlussleitung vorgesehen ist. In derartigen Ausführungsformen kann der Drucksensor 11 beispielsweise ein miniaturisierter Einweg-Drucksensor sein, welcher z.B. fest mit dem Patienteninterfacekörper 2' verbaut ist, oder der Patienteninterfacekörper 2' bzw. die Unterdruckkavität 20 sowieso der Drucksensor 11 weisen eine zumindest für den Drucksensor 11 zerstörungsfrei lösbare fluidische Schnittstelle auf.

Die Unterdruckschnittstelle 13 sowie die Drucksensorschnittstelle 14 können sich anstatt an einem Gehäuse der Unterdruckvorrichtung 1 auch an der Schnittstelle zum Patienteninterfacekörper 2' befinden. In diesem Fall können die Unterdruckverbindungsleitung 22 und die Drucksensorverbindungsleitung 23 ganz oder teilweise Teil der Unterdruckeinrichtung 1 sein. Die fluidischen Schnittstellen 13, 14 können mittels separater fluidischer Koppler, beispielsweise separater fluidischer Steckverbinder, realisiert werden, oder in einen gemeinsamen fluidischen Koppler oder Steckverbinder integriert sein.

Der Aufbau, insbesondere der fluidische Aufbau, der Unterdruckvorrichtung 1, kann ferner modifiziert werden und insbesondere weitere Komponenten umfassen. So kann die Ventilanordnung 16 gemäss Figur 3 weitere Ventile umfassen und weitere fluidische Konfigurationen ermöglichen. So kann beispielsweise vorgesehen werden, den Unterdruckgenerator 10 mit der Umgebung fluidisch zu verbinden. Ferner kann vorgesehen werden, die Unterdruckanschlussleitung 22 z.B. gemeinsam mit dem angeschlossenen zweiten Drucksensor 15 fluidisch abzuschliessen bzw. fluidisch zu isolieren. Ferner kann ein Unterdruckreservoir mit einem Volumen in einem Bereich von z.B. einem Liter vorgesehen kann, welches mittels der Ventilanordnung mit der Unterdruck-Versorgungsleitung 22 und/oder dem Unterdruckgenerator 10 verbindbar ist. Ein solches Unterdruckreservoir dient insbesondere der fluidischen Pufferung und kann ferner an Stelle und in grundsätzlich gleicher Funktion wie der Unterdruckgenerator zum Absaugen kleiner Luftmengen eingesetzt werden, beispielsweise im Falle einer kleineren und kurzzeitigen Leckage der Unterdruckkavität 20. Der Aufbau eines Unterdrucks im optionalen Unterdruckreservoir erfolgt vorteilhaft mittels des Unterdruckgenerators 10.

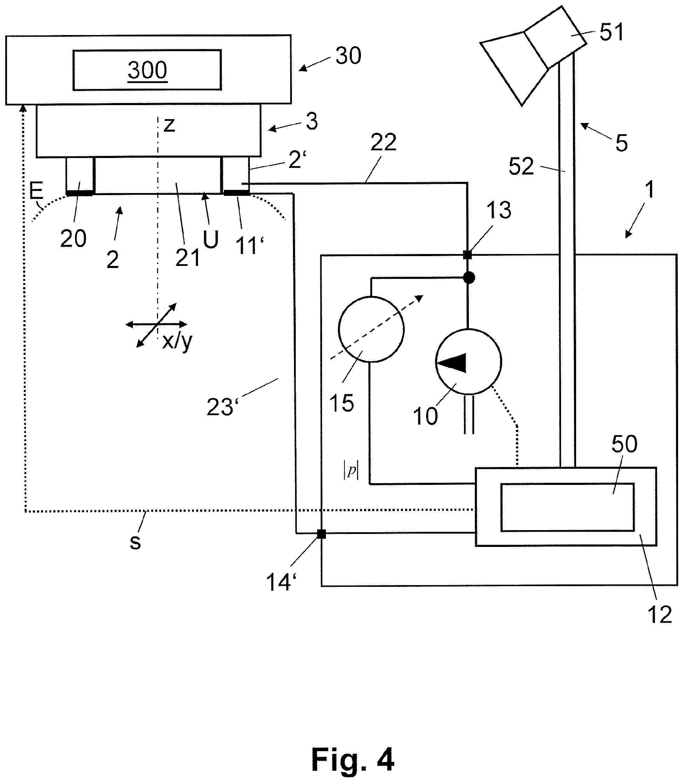

Figur 4 stellt eine weitere Ausführungsform einer ophthalmologischen Anordnung mit einer weiteren Ausführungsform der Unterdruckvorrichtung 1 und einer weiteren Ausführungsform des Patienteninterfaces 2 in einer schematischen funktionellen Darstellung und in operativ gekoppeltem Zustand dar. Soweit nicht nachfolgend erwähnt, können die Unterdruckvorrichtung 1 und das Patienteninterface 2 nach Figur 4 analog zur Darstellung gemäss Figur 1 aufgebaut sein und entsprechende Funktionalität besitzen.

In der Ausführungsform gemäß Figur 4 ist der fluidische Drucksensor 11 durch einen Kontaktdrucksensor 11' ersetzt, welcher im Patienteninterface 2 angeordnet ist. Der Kontaktdrucksensor 11' ist zur Messung des Kontraktdrucks zwischen dem Patienteninterface 2 und dem Patientenauge E ausgelegt und beispielhaft ringförmig in die dem Patientenauge E zugewandten Seite einer Wandung des Saugrings integriert. Alternativ können auch mehrere isolierte Kontaktdrucksensoren entlang des Umfangs des Saugrings oder auch nur ein einzelner isolierter Kontaktdrucksensor vorgesehen sein.

Die fluidische Drucksensorverbindungsleitung 23 der Figur 1 ist in dieser Form durch eine elektrische Drucksensorverbindungsleitung 23' ersetzt, über welche der Kontaktdrucksensor 11' mit der Unterdruckvorrichtung 1 funktionell elektrisch gekoppelt wird. Entsprechend ist statt der fluidischen vorrichtungsseitigen Drucksensorschnittstelle 14 gemäß Figur 1 eine elektrische vorrichtungsseitige Drucksensorschnittstelle 14', z. B. in Form eines elektrischen Steckverbinders, vorgesehen. Ein fluidischer Drucksensor 11 gemäß Figur 1 kann optional zusätzlich vorgesehen sein.

Wie in den Figuren 1, 3 und 4 schematisch illustriert ist, ist die Steuereinheit 12 respektive die Verarbeitungseinheit 50 über eine (elektrische) Steuerleitung mit dem Unterdruckgenerator 10 und über (elektrische) Signalleitungen mit den Drucksensoren 11, 15 respektive den Kontaktdrucksensoren 11' verbunden.

Zur Kopplung des Patienteninterfaces 2 an das Patientenauge E wird der Unterdruckgenerator 10 durch die Steuereinheit 12 angesteuert bzw. in Betrieb gesetzt, so dass die in der Unterdruckkavität 20 ursprünglich vorhandene Luft zumindest teilweise abgesaugt wird. Der funktionell separat mit der Unterdruckkavität 20 gekoppelte Drucksensor 11 misst den effektiv in der Unterdruckkavität 20 vorhandenen Druck unabhängig von der Unterdruckverbindungsleitung 22 (alternativ messen ein oder mehrere Kontaktdrucksensoren 11' den Kontraktdruck).

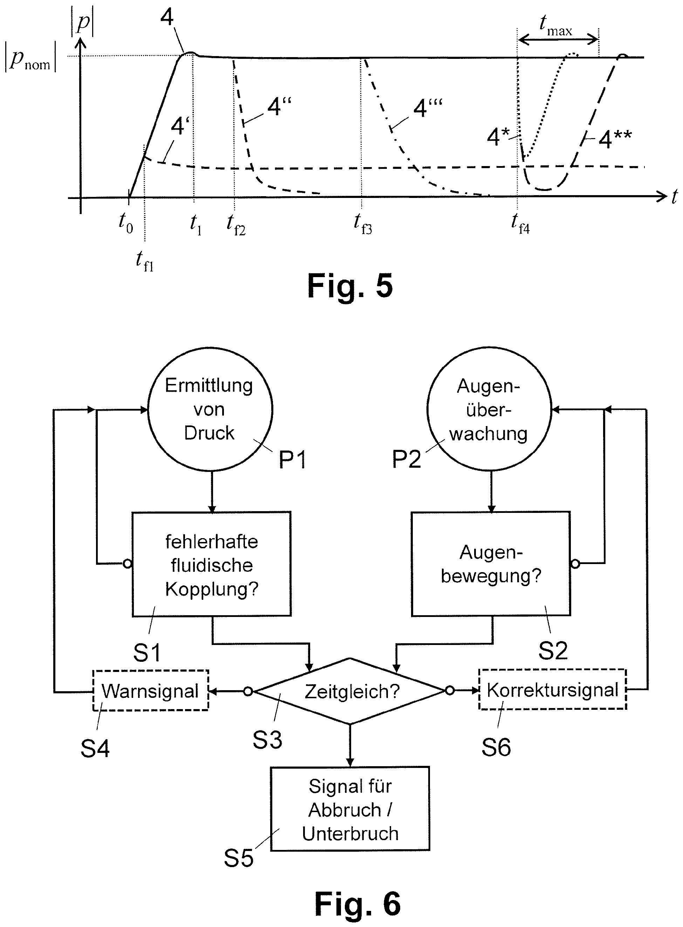

In Figur 5 stellt die mit dem Bezugszeichen 4 gekennzeichnete Kurve den Betrag des Unterdrucks *p*, so wie er vom Drucksensor 11 gemessen wird, als Funktion der Zeit *t* für eine korrekte Fixierung des Patienteninterfaces 2 auf dem Patientenauge E dar. Dabei entspricht ein Wert von *p* = 0 dem Umgebungsdruck und ein zunehmender Unterdruck (abnehmender Absolutdruck) einem ansteigenden Kurvenverlauf.

In der zum Zeitpunkt *t*₀ beginnenden Absaugphase wird in der Unterdruckkavität 20 vorhandene Luft durch den Unterdruckgenerator 10 durch die Unterdruckverbindungsleitung 22 abgesaugt, wodurch der Unterdruck ansteigt. Bei einem dem vorgesehenen Betriebszustand entsprechenden Sollunterdruck von z. B. *p*ₙₒₘ = -400mbar schaltet die Steuereinheit 12 zum Zeitpunkt *t*₁ in bekannter Weise in einen stationären Haltebetrieb um, bei welchem der Unterdruck in der Unterdruckkavität 20 im Wesentlichen konstant gehalten wird. Dieser Zustand bleibt erhalten, bis gewollt ein Druckausgleich mit der Umgebung hergestellt und so das Patienteninterface 2 von der Hornhaut des Patientenauges E gelöst wird.

Der beim Absaugen der Luft aus dem Unterdruckkavität 20 entstehende Luftstrom aus der Unterdruckkavität 20 heraus in Richtung der Unterdruckvorrichtung 1 bzw. des Unterdruckgenerators 10 bewirkt zudem eine Entfernung etwaiger vorhandener Flüssigkeitströpfchen, Resten von Sterilabdeckfolie etc. aus dem Bereich der Kopplung zwischen Drucksensorverbindungsleitung 23 und Unterdruckkavität 20.

Die Kurve 4' in Figur 5 zeigt schematisch den durch den Drucksensor 11 gemessenen Druck *p*, wenn zu einem ersten Fehlerzeitpunkt Zeitpunkt *t*_{f1} ein Pseudovakuum gemäss der zuvor beschriebenen Art entsteht. Ein in der Unterdruckvorrichtung 1 an die Unterdruckverbindungsleitung 22 angeschlossener Drucksensor 15, wie er nach dem Stand der Technik typischerweise vorhanden und bei der in Figur 1 gezeigten Unterdruckvorrichtung 1 optional ist, kann das Abknicken der Unterdruckverbindungsleitung 22 nicht detektieren, da zwischen dem Ort des Abknickens und der Unterdruckvorrichtung 1 bzw. dem Unterdruckgenerator 10 weiter ein Unterdruck vorhanden ist bzw. auch weiter aufgebaut wird, und so vom Drucksensor 15 ein Unterdruck gemessen wird, der jedoch in der Unterdruckkavität 20 nicht vorhanden ist. Aufgrund der unmittelbaren und von der Unterdruckverbindungsleitung 22 unabhängigen Kopplung des Drucksensors 11 an die Unterdruckkavität 20 misst der Drucksensor 11 dagegen den in der Unterdruckkavität 20 effektiv vorhanden Druck. Entsprechend steigt der vom Drucksensor 11 gemessene Unterdruck nach dem Abknicken der Unterdruckverbindungsleitung 20 nicht weiter an, obwohl der Unterdruckgenerator 10 weiterarbeitet. Der Unterdruck in der Unterdruckkavität 20 bleibt nach dem Auftreten des Pseudovakuums (auf zu geringem Niveau) im Wesentlichen konstant oder fällt aufgrund von Elastizität und/oder gegebenenfalls vorhandenen Leckagen wieder ab, so dass ein Druckausgleich mit der Umgebung stattfindet.

Die Kurve 4" in Figur 5 stellt den vom Drucksensor 11 gemessenen Druck für den Fall dar, dass der Unterdruck in der Unterdruckkavität 20 (nach einem zunächst korrekt erfolgten Aufbau des Unterdrucks und einer korrekten Fixierung des Patienteninterfaces 2 auf dem Patientenauge E) zu einem zweiten Fehlerzeitpunkt *t*_{f2} während des stationären Betriebs abfällt. Dies ist zum Beispiel der Fall, wenn sich das Patienteninterface 2 vom Patientenauge E kurzfristig und teilweise löst, was in ungünstigen Fällen, z. B. aufgrund einer erforderlichen Bewegung des Patienteninterfaces durch den Augenarzt oder auch eine Bewegung des Patienten selbst, erfolgen kann. In diesem Fall findet ein zumindest teilweiser Druckausgleich mit der Umgebung und damit ein Abfall des Unterdrucks in der Unterdruckkavität 20 statt. Grundsätzlich kann dieser Druckabfall durch den Drucksensor 15 detektiert werden und der Unterdruckgenerator 10 kann den korrekten Unterdruck mindestens im Fall einer nur kurzzeitigen Leckage wiederherstellen. Ist jedoch die Unterdruckverbindungsleitung 22 selbst abgeknickt oder aus anderem Grunde nicht durchgängig, wird der Abfall des Unterdrucks durch den Drucksensor 15 nicht erkannt. Bei einer nur teilweise durchgängigen Unterdruckverbindungsleitung 22 erhöht sich zumindest die Regelverzögerung beim Ausregeln des Druckabfalls, so dass eine sichere Fixierung des Patienteninterfaces 2 auf dem Patientenauge E unter Umständen ebenfalls nicht mehr gegeben ist oder zumindest nicht gewährleistet werden kann. Der unmittelbar an die Unterdruckkavität 20 angeschlossene Drucksensor 11 stellt den Abfall des Unterdrucks gemäss Kurve 4" jedoch korrekt fest.

Die Kurve 4'" in Figur 5 stellt den Fall dar, dass (nach einem zunächst korrekt erfolgten Aufbau des Unterdrucks und einer korrekten Fixierung des Patienteninterfaces 2 auf dem Patientenauge E) der Unterdruck zu einem dritten Fehlerzeitpunkt *t*_{f3} (im Vergleich zum in Kurve 4" dargestellten Verlauf) langsam abfällt, was z. B. durch eine geringfügige Leckage oder Undichtigkeit eines fluidischen Verbinders bedingt werden kann.

Die Kurven 4* und 4** in Figur 5 stellen Fälle dar, in denen (nach einem zunächst korrekt erfolgten Aufbau des Unterdrucks und einer korrekten Fixierung des Patienteninterfaces 2 auf dem Patientenauge E) der Unterdruck zu einem vierten Fehlerzeitpunkt *t*_{f4} kurzzeitig und temporär abfällt, was z. B. durch eine vorüberhegende Blockierung der Unterdruckverbindungsleitung 22 - aber auch der Drucksensorverbindungsleitung 23 - verursacht werden kann. Wie im Fall der der Kurve 4* ersichtlich ist, wird der dem vorgesehenen Betriebszustand entsprechende Sollunterdruck *p*ₙₒₘ innerhalb einer Zeitdauer wieder aufgebaut, der vor (respektive innerhalb) einer vorgegebenen maximalen Zeitdauer *t*ₘₐₓ liegt. Im Fall der Kurve 4** hingegen, wird der Sollunterdruck *p*ₙₒₘ erst nach Ablauf der maximalen Zeitdauer *t*ₘₐₓ wieder erreicht.

Figur 6 illustriert schematisch Prozesse P1, P2 und Schritte S1, S2, S3, S4, S5 zur Überwachung des ophthalmologischen Patienteninterfaces 2.

Im Prozess P1 erfolgt die kontinuierliche oder quasi-kontinuierlich Ermittlung des Drucks (Unterdruck) in der Unterdruckkavität 20. Abhängig von der Ausführungsvariante und Konfiguration erfolgt die Ermittlung des Drucks im Prozess P1 durch den Drucksensor 11, alternativ durch einen oder mehrere Kontaktdrucksensoren 11', optional ergänzend durch einen oder mehrere (redundante) Kontaktdrucksensoren 11', und optional ergänzend durch den (redundanten) Drucksensor 15.

Im Schritt S1, bestimmt die Verarbeitungseinheit 50 basierend auf den im Prozess P1 ermittelten Druckwerten, ob eine fehlerhafte fluidische Kopplung des Patienteninterfaces 2 respektive der Unterdruckkavität 20 vorliegt. Dabei wird der gemessene Druck kontinuierlich oder quasi-kontinuierlich mit mindestens einem Grenzunterdruck *p*ₗᵢₘᵢₜ verglichen. Dieser Grenzdruck *p*ₗᵢₘᵢₜ liegt typischerweise betragsmässig tiefer als der Sollunterdruck *p*ₙₒₘ und kann z. B. durch den Betrag des Sollunterdruck *p*ₙₒₘ abzüglich eines durch Toleranzen, Messunsicherheit etc. bestimmten Sicherheitswertes erfolgen. In der Absaugphase kann der Grenzdruck *p*ₗᵢₘᵢₜ entsprechend dem sich bei korrektem Betrieb ergebenden Druckverlauf kontinuierlich angepasst werden. Ein Fehlerfall wird dann bei Unterschreitung des Grenzdrucks *p*ₗᵢₘᵢₜ angenommen.

Alternativ oder ergänzend wird der gemessene Druck kontinuierlich oder quasi-kontinuierlich hinsichtlich eines Abfalls bzw. Nachlassen des Unterdrucks ausgewertet. Hierfür können zahlreiche grundsätzlich bekannte Verfahren der Signalverarbeitung und/oder Statistik zum Einsatz kommen, beispielsweise die Bestimmung und Auswertung der Steigung und/oder weiterer Kennwerte einer durch eine Interpolation von Messwerten gebildeten Funktion.

Umfasst die Unterdruckvorrichtung 1 zusätzlich den optionalen zweiten Drucksensor 15 wird alternativ oder ergänzend die Detektion einer fehlerhaften fluidischen Kopplung des Patienteninterfaces 2 durch einen Vergleich bzw. eine gemeinsame Auswertung der durch den Drucksensor 11 und den zweiten Drucksensor 15 ermittelten Drücke ausgeführt. Im Falle einer korrekten Kopplung des Patienteninterfaces 2 und ohne das Vorliegen eines Fehlerzustandes sind die vom Drucksensor 11 und dem zweiten Drucksensor 15 gemessenen Drücke mindestens im stationären Betriebszustand im Wesentlichen gleich und die Sensoren 11, 15 damit redundant. Entsprechend kann die Detektion einer fehlerhaften oder ungenügenden fluidischen Kopplung des Patienteninterfaces 2 die Detektion einer Abweichung zwischen den durch den Drucksensor 11 bzw. den zweiten Drucksensor 15 ermittelten Drücken umfassen. Das Ermitteln der Abweichung kann dabei mittels grundsätzlich bekannter Verfahren der Signalverarbeitung und/oder Statistik erfolgen und z.B. einer Ermittlung und Auswertung einer Differenz der ermittelten Drücke und den Vergleich mit einer zulässigen Höchstdifferenz umfassen, welche sich typischerweise aus Toleranzen und Messunsicherheiten bestimmt. Alternativ oder ergänzend zu einer Differenzbildung kann das Feststellen einer fehlerhaften oder ungenügenden Kopplung beispielsweise das Ermitteln und Auswerten einer Korrelation der durch den Drucksensor 11 und den zweiten Drucksensor 15 ermittelten Drücke in Funktion der Zeit t umfassen.

In einer Ausführungsvariante berücksichtigt die Steuervorrichtung 12 respektive die Verarbeitungseinheit 50 bei der Detektion einer fehlerhaften fluidischen Kopplung des Patienteninterfaces 2 die Zeitdauer, während der ein Druckabfall festgestellt wird, um eine ophthalmologische Laserbehandlung nicht unnötig wegen eines kurzzeitigen, temporären Druckabfalls zu unterbrechen, welcher keine störende Ablösung oder Verschiebung des Patienteninterfaces 2 vom respektive relativ zum Patientenauge E bewirkt. Dabei wird ein kurzzeitiger, temporärer Druckabfall, der innerhalb einer vorgegebenen maximalen Zeitdauer *t*ₘₐₓ wieder über dem erforderlichen Grenzdruck *p*ₗᵢₘᵢₜ liegt nicht als fehlerhafte fluidische Kopplung des Patienteninterfaces 2 behandelt, zumindest nicht, wenn solche kurzzeitigen, temporären Druckabfälle sich nicht mit einer grenzüberschreitenden Häufigkeit wiederholen (Oszillationsverhalten).

Bei einer gemeinsamen Auswertung der durch den Drucksensor 11 und den zweiten Drucksensor 15 ermittelten Drücke ist zu beachten, dass der Drucksensor 11 insbesondere während des Aufbaus des Unterdrucks in der Unterdruckkavität 20 und beim Entlüften auf Druckänderungen in der Unterdruckkavität 20 schneller reagiert als der zweite Unterdrucksensor 15. Dies ergibt sich daraus, dass über die Unterdruckverbindungsleitung 22 zugleich eine Verschiebung von Luftvolumen erfolgt, während die Drucksensorverbindungsleitung 23 durch den Drucksensor 11 fluidisch abgeschlossen ist.

Durch eine gemeinsame Auswertung der durch den Drucksensor 11 und den zweiten Drucksensor 15 ermittelten Drücke ergibt sich der Vorteil, dass die Steuervorrichtung 12 respektive die Verarbeitungseinheit 50 zudem weitere Fehlerzustände detektieren kann, beispielsweise ein Pseudovakuum der Drucksensorverbindungsleitung 23 oder Defekte der Drucksensoren 11, 15, ihrer elektrischen Kontaktierung und/oder von ihnen nachgeschalteten Komponenten.

Im Prozess P2 erfolgt die kontinuierliche oder quasi-kontinuierliche (Video-) Überwachung des Patientenauges E durch die Sensorvorrichtung(en) 51 des Bewegungsdetektors 5.

Im Schritt S2, detektiert die Verarbeitungseinheit 50 Bewegungen des Patientenauges E basierend auf der im Prozess P2 erfolgten Überwachung (Videoüberwachung, Videoerfassung, OCT-Vermessung, Triangulation, etc.) respektive auf den im Prozess P2 durch die Sensorvorrichtung(en) 51 gelieferten Messsignale (z.B. Videosignale).

Im Schritt S3, untersucht die Verarbeitungseinheit 50, ob gleichzeitig mit einer detektierten fehlerhaften fluidischen Kopplung des Patienteninterfaces 2 eine detektierte Bewegung des Patientenauges E vorliegt. Wenn dies *nicht* der Fall ist, wird im Prozess P1 die laufende Ermittlung des Drucks in der Unterdruckkavität 20 sowie im Prozess P2 die laufende Überwachung des Patientenauges E fortgeführt, ohne dass die ophthalmologische Behandlung durch die ophthalmologische Behandlungsvorrichtung 30 unterbrochen oder abgebrochen wird.

Im optionalen Schritt S4 erzeugt die Verarbeitungseinheit 50 ein Warnsignal, das anzeigt, dass eine fehlerhafte fluidische Kopplung des Patienteninterfaces 2 detektiert wurde. Zu diesem Zweck umfasst die Steuereinheit 12 eine Alarmierungsvorrichtung oder ist mit einer (nicht dargestellten) Alarmierungsvorrichtung verbunden, welche eingerichtet ist, aufgrund des Warnsignals einen optischen und/oder akustischen Alarm zu erzeugen.

Im optionalen Schritt S6 erzeugt die Verarbeitungseinheit 50 nach erfolgter Augendetektion ein Korrektursignal für die ophthalmologische Behandlungsvorrichtung 30 respektive das Lasersystem 300, wenn keine fehlerhafte fluidische Kopplung der Unterdruckkavität 20 detektiert wird. Das Korrektursignal ermöglicht der ophthalmologischen Behandlungsvorrichtung 30 respektive dem Lasersystem 300 den Laserstrahl respektive dessen Fokus unter Berücksichtigung der detektierten Augenbewegung und der Sollposition (gemäss Steuerdaten der ophthalmologischen Behandlung) neu auszurichten respektive zu positionieren und die ophthalmologische Behandlung fortzusetzen, wenn keine fehlerhafte fluidische Kopplung der Unterdruckkavität 20 detektiert wird. Das Korrektursignal umfasst eine Angabe der detektierten Augenbewegung, beispielsweise die aktuelle (Ist-) Lage des Patientenauges E oder einen Verschiebungsvektor, der die Bewegung des Patientenauges E, ausgehend von seiner Ausgangslage, vor der detektierten Augenbewegung, zu seiner Ist-Lage, nach der detektierten Augenbewegung, definiert und der ophthalmologischen Behandlungsvorrichtung 30 respektive dem Lasersystem 300 eine Korrektur mit Neuausrichtung des Laserstrahls respektive Neupositionierung von dessen Fokus ermöglicht.

Wenn gleichzeitig eine fehlerhafte fluidische Kopplung des Patienteninterfaces 2 und eine Augenbewegung des Patienten festgestellt wird, erzeugt die Verarbeitungseinheit 50 im Schritt S5 ein Steuersignal s zum Abbrechen der ophthalmogischen Behandlung, die durch die ophthalmologische Behandlungsvorrichtung 30 durchgeführt wird. Wie in den Figuren 1 und 4 schematisch dargestellt ist, wird das Steuersignal s von der Steuereinheit 12 respektive der Verarbeitungseinheit 50 über eine Signalleitung der ophthalmologischen Behandlungsvorrichtung 30 zugeführt. Zu diesem Zweck ist die Steuereinheit 12 respektive die Verarbeitungseinheit 50 mit der ophthalmologischen Behandlungsvorrichtung 30 verbunden oder in der ophthalmologischen Behandlungsvorrichtung 30 angeordnet, beispielsweise im Applikationskopf 3. In der ophthalmologischen Behandlungsvorrichtung 30 wird die ophthalmogische Behandlung aufgrund des eingehenden Steuersignals dadurch unterbrochen respektive abgebrochen, dass beispielsweise das Lasersystem 300 ausgeschaltet wird und/oder der Laserstrahl durch Bewegen einer Blende oder eines Shutters unterbrochen wird. Zudem erzeugt die die Steuereinheit 12 respektive die Verarbeitungseinheit 50 ein Alarmsignal, wie obenstehend im Zusammenhang des Warnsignals von Schritt S4 beschrieben wurde.

## Patentansprüche

1. Unterdruckvorrichtung (1) zum Fixieren eines ophthalmologischen Patienteninterfaces (2) auf einem Patientenauge (E), die Unterdruckvorrichtung (1) umfassend:
einen Unterdruckgenerator (10) und eine Unterdruckschnittstelle (13) zur fluidischen Kopplung des Unterdruckgenerators (10) an eine Unterdruckkavität (20) des einen Patienteninterfaces (2);
einen an das eine Patienteninterface (2) fluidisch ankoppelbaren internen Drucksensor (11) und/oder eine Druckmessschnittstelle (14') zur signaltechnischen Ankopplung eines externen Drucksensors (11') des einen Patienteninterfaces (2);
einen Bewegungsdetektor (5), der eingerichtet ist, Bewegungen des Patientenauges (E) zu detektieren; und
eine Steuereinheit (12), die eingerichtet ist, aufgrund eines vom angekoppelten Drucksensor (11, 11') ermittelten Drucks eine fehlerhafte fluidische Kopplung der Unterdruckkavität (20) zu detektieren,
**dadurch gekennzeichnet, dass** die Steuereinheit (12) zudem eingerichtet ist, ein Steuersignal (s) zum Abbrechen einer durch eine ophthalmologische Behandlungsvorrichtung (30) durchgeführten ophthalmogischen Behandlung zu erzeugen, wenn gleichzeitig mit der detektierten fehlerhaften fluidischen Kopplung der Unterdruckkavität (20) vom Bewegungsdetektor (5) eine Augenbewegung detektiert wird.

2. Unterdruckvorrichtung (1) nach Anspruch 1, **dadurch gekennzeichnet, dass** die Steuereinheit (12) eingerichtet ist, ohne Unterbruch der ophthalmogischen Behandlung ein Warnsignal zu erzeugen, wenn während einer detektierten fehlerhaften fluidischen Kopplung der Unterdruckkavität (20) keine Augenbewegung detektiert wird.

3. Unterdruckvorrichtung (1) nach einem der Ansprüche 1 oder 2, **dadurch gekennzeichnet, dass** der Bewegungsdetektor (5) einen Videosensor (51) und eine mit dem Videosensor (51) verbundene Verarbeitungseinheit (50) umfasst, welche eingerichtet ist, die Augenbewegungen aufgrund von vom Videosensor (51) gelieferten Videosignalen zu detektieren.

4. Unterdruckvorrichtung (1) nach einem der Ansprüche 1 bis 3, **dadurch gekennzeichnet, dass** der Bewegungsdetektor (5) eingerichtet ist, Augenbewegungen zu detektieren, welche das Patientenauge (E) relativ zu einer starren Beobachtungsachse (z) bewegen, in einer ersten Bewegungsrichtung in einer zur Beobachtungsachse (z) normalen Ebene (x/y), und in einer zweiten Bewegungsrichtung entlang der Beobachtungsachse (z).

5. Unterdruckvorrichtung (1) nach einem der Ansprüche 1 bis 4, **dadurch gekennzeichnet, dass** die Steuereinheit (12) eingerichtet ist, das Steuersignal (s) zum Abbrechen der ophthalmogischen Behandlung zu erzeugen, wenn vom Bewegungsdetektor (5) eine Augenbewegung detektiert wird, die über einem definierten Toleranzschwellwert liegt.

6. Unterdruckvorrichtung (1) nach einem der Ansprüche 1 bis 5, **dadurch gekennzeichnet, dass** die Unterdruckvorrichtung (1) eine mit dem internen Drucksensor (11) fluidisch verbundene Drucksensorschnittstelle (14) umfasst, die eingerichtet ist, den internen Drucksensor (11) separat zu der Unterdruckschnittstelle (13) fluidisch mit dem einen Patienteninterface (2) zu koppeln.

7. Unterdruckvorrichtung (1) nach einem der Ansprüche 1 bis 6, **dadurch gekennzeichnet, dass** die Druckmessschnittstelle (14') eingerichtet ist zur signaltechnischen Ankopplung eines an dem einen Patienteninterface (2) angeordneten externen Kontaktdrucksensors (11').

8. Unterdruckvorrichtung (1) nach einem der Ansprüche 1 bis 7, **dadurch gekennzeichnet, dass** die Steuereinheit (12) eingerichtet ist, die fehlerhafte fluidische Kopplung der Unterdruckkavität (20) durch Detektion einer Abweichung zwischen dem mittels des angekoppelten Drucksensors (11, 11') ermittelten Drucks und einem Referenzdruck, und/oder durch Detektion eines Abfalls des Drucks in Funktion der Zeit, zu detektieren.

9. Unterdruckvorrichtung (1) nach einem der Ansprüche 1 bis 8, **dadurch gekennzeichnet, dass** die Unterdruckvorrichtung (1) einen mit der Unterdruckschnittstelle (13) fluidisch gekoppelten und mit der Steuereinheit (12) verbundenen zweiten Drucksensor (15) umfasst, und dass die Steuereinheit (12) eigerichtet ist, die fehlerhafte fluidische Kopplung der Unterdruckkavität (20) durch Vergleichen des ermittelten Drucks mit einem vom zweiten Drucksensor (15) ermittelten zweiten Druck zu detektieren.

10. Unterdruckvorrichtung (1) nach einem der Ansprüche 1 bis 9, **dadurch gekennzeichnet, dass** der Bewegungsdetektor (5) eine Sensorvorrichtung (51) umfasst, welche eingerichtet ist, die Augenbewegungen aufgrund von Distanzveränderungen des Patientenauges (E) relativ zu dem einen Patienteninterface (2) zu detektieren.

11. Unterdruckvorrichtung (1) nach einem der Ansprüche 1 bis 10, **dadurch gekennzeichnet, dass** die Steuereinheit (12) eingerichtet ist, aufgrund einer detektierten Augenbewegung ein Korrektursignal zur Repositionierung und Fortführung der ophthalmogischen Behandlung zu erzeugen, wenn eine Beendigung der Augenbewegung und keine fehlerhafte fluidische Kopplung der Unterdruckkavität (20) detektiert wird.

12. Verfahren zum Überwachen eines ophthalmologischen Patienteninterfaces (2), das mittels eines, durch einen Unterdruckgenerator in einer Unterdruckkavität (20) des Patienteninterfaces (2) hergestellten, Unterdrucks auf einem Patientenauge (E) fixiert ist, das Verfahren umfassend:
Ermitteln (P1) eines Drucks mittels eines fluidisch an das Patienteninterface (2) angekoppelten Drucksensors (11) und/oder eines Kontaktdrucksensors (11') des Patienteninterfaces (2);
Detektieren (S2) von Bewegungen des Patientenauges (E) mittels eines Bewegungsdetektors (5); und
Detektieren (S1) durch eine Steuereinheit (12) einer fehlerhaften fluidischen Kopplung der Unterdruckkavität (20) an den Unterdruckgenerator (10) aufgrund des ermittelten Drucks;
**gekennzeichnet durch** Erzeugen (S5) durch die Steuereinheit (12) eines Steuersignals (s) zum Abbrechen einer durch eine ophthalmologische Behandlungsvorrichtung (30) durchgeführten ophthalmogischen Behandlung, wenn gleichzeitig mit der detektierten fehlerhaften fluidischen Kopplung der Unterdruckkavität (20) vom Bewegungsdetektor (5) eine Augenbewegung detektiert wird.

13. Verfahren nach Anspruch 12, **dadurch gekennzeichnet, dass** die Steuereinheit (12) ohne Unterbruch der ophthalmogischen Behandlung ein Warnsignal erzeugt, wenn während einer detektierten fehlerhaften fluidischen Kopplung der Unterdruckkavität (20) keine Augenbewegung detektiert wird.

14. Verfahren nach einem der Ansprüche 12 oder 13, **gekennzeichnet durch** Videoerfassung (P2) des Patientenauges (E) durch einen Videosensor (51), und Detektieren (S2) der Augenbewegungen durch die Steuereinheit (12) aufgrund der Videoerfassung.

15. Verfahren nach einem der Ansprüche 12 bis 14, **dadurch gekennzeichnet, dass** die Steuereinheit (12) das Steuersignal (s) zum Abbrechen der ophthalmogischen Behandlung erzeugt, wenn vom Bewegungsdetektor (5) eine Augenbewegung detektiert wird, die über einem definierten Toleranzschwellwert liegt.

16. Ophthalmologische Behandlungsvorrichtung (30) umfassend ein Lasersystem (300) zur ophthalmologischen Behandlung eines Patientenauges (E), einen Applikationskopf (3), und ein Patienteninterface (2) zum Anbringen des Applikationskopfes (3) auf dem Patientenauge (E), wobei die ophthalmologische Behandlungsvorrichtung (30) zum Fixieren des ophthalmologischen Patienteninterfaces (2) auf dem Patientenauge (E) eine Unterdruckvorrichtung (1) gemäss einem der Ansprüche 1 bis 10 umfasst.

## Claims

1. Vacuum device (1) for affixing an ophthalmological patient interface (2) on a patient's eye (E), said vacuum device (1) comprising:
a vacuum generator (10) and a vacuum interface (13) for fluidic coupling of the vacuum generator (10) on a vacuum cavity (20) of the patient interface (2);
an internal pressure sensor (11) that is fluidically coupleable to the patient interface (2) and/or a pressure-measuring interface (14') for a signal-coupling of an external pressure sensor (11') of the patient interface (2);
a movement detector (5) which is configured to detect movements of the patient's eye (E); and
a control unit (12) which is configured to detect a faulty fluidic coupling of the vacuum cavity (20) on the basis of a pressure that is detected by the coupled pressure sensor (11, 11'),
**characterized in that** the control unit (12) is furthermore configured to produce a control signal (s) for interrupting an ophthalmological treatment that is carried out by an ophthalmological treatment device (30) if, simultaneously to the detected faulty fluidic coupling of the vacuum cavity (20), an eye movement is detected by the movement detector (5).

2. Vacuum device (1) according to Claim 1, **characterized in that** the control unit (12) is configured to produce a warning signal without interrupting the ophthalmological treatment if no eye movement is detected during a detected faulty fluidic coupling of the vacuum cavity (20).

3. Vacuum device (1) according to either of Claims 1 and 2, **characterized in that** the movement detector (5) comprises a video sensor (51) and a processing unit (50) that is connected to the video sensor (51) and configured to detect the eye movements on the basis of video signals supplied by the video sensor (51).

4. Vacuum device (1) according to any one of Claims 1 to 3, **characterized in that** the movement detector (5) is configured to detect eye movements which move the patient's eye (E) relative to a static observation axis (z), in a first movement direction in a plane (x/y) that is normal to the observation axis (z) and in a second movement direction along the observation axis (z).

5. Vacuum device (1) according to any one of Claims 1 to 4, **characterized in that** the control unit (12) is configured to produce the control signal (s) for interrupting the ophthalmological treatment if the movement detector (5) detects an eye movement that lies over a defined tolerance threshold.

6. Vacuum device (1) according to any one of Claims 1 to 5, **characterized in that** the vacuum device (1) comprises a pressure sensor interface (14) that is fluidically connected to the internal pressure sensor (11), said pressure sensor interface being configured to fluidically couple the internal pressure sensor (11) to the patient interface (2), separately from the vacuum interface (13).

7. Vacuum device (1) according to any one of Claims 1 to 6, **characterized in that** the pressure-measuring interface (14') is configured for signal-technical coupling of an external contact pressure sensor (11') that is arranged at the patient interface (2) .

8. Vacuum device (1) according to any one of Claims 1 to 7, **characterized in that** the control unit (12) is configured to detect the faulty fluidic coupling of the vacuum cavity (20) by detecting a deviation between the pressure that is detected by means of the coupled pressure sensor (11, 11') and a reference pressure, and/or by detecting a drop in the pressure as a function of time.

9. Vacuum device (1) according to any one of Claims 1 to 8, **characterized in that** the vacuum device (1) comprises a second pressure sensor (15) that is fluidically coupled to the vacuum interface (13) and connected to the control unit (12) and **in that** the control unit (12) is configured to detect the faulty fluidic coupling of the vacuum cavity (20) by comparing the detected pressure with a second pressure that is detected by the second pressure sensor (15).

10. Vacuum device (1) according to any one of Claims 1 to 9, **characterized in that** the movement detector (5) comprises a sensor device (51) that is configured to detect the eye movements on the basis of changes in the distance of the patient's eye (E) relative to the patient interface (2).

11. Vacuum device (1) according to any one of Claims 1 to 10, **characterized in that** the control unit (12) is configured to produce a correction signal for repositioning and continuing the ophthalmological treatment on the basis of a detected eye movement if a termination of the eye movement and no faulty fluidic coupling of the vacuum cavity (20) are detected.

12. Method of monitoring an ophthalmological patient interface (2) which is affixed on a patient's eye (E) by means of a vacuum that is produced by a vacuum generator (10) in a vacuum cavity (20) of the patient interface (2), said method comprising the following steps:
detecting (P1) a pressure by means of a pressure sensor (11) that is fluidically coupled to the patient interface (2) and/or by means of a contact pressure sensor (11') of the patient interface (2) ;
detecting (S2) movements of the patient's eye (E) by means of a movement detector (5); and
detecting (S1) a faulty fluidic coupling of the vacuum cavity (20) at the vacuum generator (10) by way of a control unit (12) on the basis of the detected pressure;
**characterized by** producing (S5), by way of the control unit (12), a control signal (s) for interrupting an ophthalmological treatment that is carried out by an ophthalmological treatment device (30) if, simultaneously to the detected faulty fluidic coupling of the vacuum cavity (20), an eye movement is detected by the movement detector (5).

13. Method according to Claim 12, **characterized in that** the control unit (12) produces a warning signal without interrupting the ophthalmological treatment if no eye movement is detected during a detected faulty fluidic coupling of the vacuum cavity (20).

14. Method according to either of Claims 12 and 13, **characterized by** a video capture (P2) of the patient's eye (E) by a video sensor (51) and detecting (S2) of the eye movements by the control unit (12) on the basis of the video capture.

15. Method according to any one of Claims 12 to 14, **characterized in that** the control unit (12) produces the control signal (s) for interrupting the ophthalmological treatment if the movement detector (5) detects an eye movement that lies above a defined tolerance threshold.

16. Ophthalmological treatment device (30) comprising a laser system (300) for the ophthalmological treatment of a patient's eye (E), an application head (3) and a patient interface (2) for attaching the application head (3) to the patient's eye (E), wherein the ophthalmological treatment device (30) comprises a vacuum device (1) according to any one of Claims 1 to 10 for affixing the ophthalmological patient interface (2) on the patient's eye (E).

## Revendications

1. Dispositif de dépression (1) pour la fixation d'une interface ophtalmologique de patient (2) sur un œil de patient (E), le dispositif de dépression (1) comprenant
un générateur de dépression (10) et une interface de dépression (13) pour le couplage fluidique du générateur de dépression (10) avec une cavité de dépression (20) de l'interface de patient (2) ;
un capteur de pression interne (11) pouvant être accouplé de manière fluidique avec l'interface de patient (2) et/ou une interface de mesure de pression (14') pour le couplage, par technique de signalisation, d'un capteur de pression externe (11') de l'interface de patient (2) ;
un détecteur de mouvement (5) conçu pour détecter des mouvements de l'œil du patient (E) ; et
une unité de commande (12) qui est conçue pour détecter un couplage fluidique erroné de la cavité de dépression (20) en raison d'une pression déterminée par le capteur de pression (11, 11') accouplé,
**caractérisé en ce que** l'unité de commande (12) est en outre conçue pour générer un signal de commande (s) pour l'interruption d'un traitement ophtalmologique réalisé par un dispositif de traitement ophtalmologique (30) si, de manière simultanée au couplage fluidique erroné détecté de la cavité de dépression (20), un mouvement de l'œil est détecté par le détecteur de mouvement (5).

2. Dispositif de dépression (1) selon la revendication 1, **caractérisé en ce que** l'unité de commande (12) est conçue pour générer un signal d'alarme sans interruption du traitement ophtalmologique si aucun mouvement de l'œil n'est détecté pendant un couplage fluidique erroné détecté de la cavité de dépression (20) .

3. Dispositif de dépression (1) selon l'une des revendications 1 et 2, **caractérisé en ce que** le détecteur de mouvement (5) comprend un capteur vidéo (51) et une unité de traitement (50) reliée au capteur vidéo (51), laquelle est conçue pour détecter les mouvements de l'œil en raison de signaux vidéo délivrés par le capteur vidéo (51).

4. Dispositif de dépression (1) selon l'une des revendications 1 à 3, **caractérisé en ce que** le détecteur de mouvement (5) est conçu pour détecter des mouvements de l'œil qui font bouger l'œil du patient (E) par rapport à un axe rigide d'observation (z), dans une première direction de mouvement dans un plan (x/y) normal à l'axe d'observation (z), et dans une deuxième direction de mouvement le long de l'axe d'observation (z) .

5. Dispositif de dépression (1) selon l'une des revendications 1 à 4, **caractérisé en ce que** l'unité de commande (12) est conçue pour générer le signal de commande (s) pour interrompre le traitement ophtalmologique si un mouvement de l'œil est détecté par le détecteur de mouvement (5), lequel se situe au-dessus d'une valeur de seuil de tolérance définie.

6. Dispositif de dépression (1) selon l'une des revendications 1 à 5, **caractérisé en ce que** le dispositif de dépression (1) comprend une interface de capteur de pression (14) reliée de manière fluidique au capteur de pression interne (11), laquelle est conçue pour coupler le capteur de pression interne (11) de manière fluidique, séparément de l'interface de dépression (13), avec l'interface de patient (2).

7. Dispositif de dépression (1) selon l'une des revendications 1 à 6, **caractérisé en ce que** l'interface de mesure de pression (14') est conçue pour l'accouplement, par technique de signalisation, d'un capteur de pression de contact (11') externe disposé au niveau de l'interface de patient (2).

8. Dispositif de dépression (1) selon l'une des revendications 1 à 7, **caractérisé en ce que** l'unité de commande (12) est conçue pour détecter le couplage fluidique erroné de la cavité de dépression (20) par détection d'une divergence entre la pression déterminée au moyen du capteur de pression (11, 11') accouplé et une pression de référence, et/ou par détection d'une baisse de la pression en fonction du temps.

9. Dispositif de dépression (1) selon l'une des revendications 1 à 8, **caractérisé en ce que** le dispositif de dépression (1) comprend un deuxième capteur de pression (15) couplé de manière fluidique avec l'interface de dépression (13) et relié à l'unité de commande (12), et que l'unité de commande (12) est conçue pour détecter le couplage fluidique erroné de la cavité de dépression (20) par comparaison de la pression déterminée avec une deuxième pression déterminée par le deuxième capteur de pression (15).

10. Dispositif de dépression (1) selon l'une des revendications 1 à 9, **caractérisé en ce que** le détecteur de mouvement (5) comprend un dispositif de détection (51) conçu pour détecter les mouvements de l'œil en raison de modifications de distance de l'œil du patient (E) par rapport à l'interface de patient (2) .

11. Dispositif de dépression (1) selon l'une des revendications 1 à 10, **caractérisé en ce que** l'unité de commande (12) est conçue pour, en raison d'un mouvement de l'œil détecté, générer un signal de correction pour le repositionnement et la poursuite du traitement ophtalmologique si un achèvement du mouvement de l'œil est détecté et aucun couplage fluidique erroné de la cavité de dépression (20) n'est détecté.

12. Procédé pour surveiller une interface ophtalmologique de patient (2), laquelle est fixée, au moyen d'une dépression générée par un générateur de dépression (10) dans une cavité de dépression (20) de l'interface de patient (2), sur un œil de patient (E), le procédé comprenant :
la détermination (P1) d'une pression au moyen d'un capteur de pression (11) accouplé de manière fluidique avec l'interface de patient (2) et/ou d'un capteur de pression de contact (11') de l'interface de patient (2) ;
la détection (S2) de mouvements de l'œil du patient (E) au moyen d'un détecteur de mouvement (5) ; et
la détection (S1) par une unité de commande (12) d'un couplage fluidique erroné de la cavité de dépression (20) avec le générateur de dépression (10) en raison de la pression déterminée ;
**caractérisé par** la génération (S5) par l'unité de commande (12) d'un signal de commande (s) pour l'interruption d'un traitement ophtalmologique réalisé par un dispositif de traitement ophtalmologique (30) si, de manière simultanée au couplage fluidique erroné détecté de la cavité de dépression (20), un mouvement de l'œil est détecté par le détecteur de mouvement (5).

13. Procédé selon la revendication 12, **caractérisé en ce que** l'unité de commande (12) génère un signal d'alarme sans interruption du traitement ophtalmologique si aucun mouvement de l'œil n'est détecté pendant un couplage fluidique erroné détecté de la cavité de dépression (20).

14. Procédé selon l'une des revendications 12 et 13, **caractérisé par** capture vidéo (P2) de l'œil du patient (E) par un capteur vidéo (51) et détection (S2) des mouvements de l'œil par l'unité de commande (12) en raison de la capture vidéo.

15. Procédé selon l'une des revendications 12 à 14, **caractérisé en ce que** l'unité de commande (12) génère le signal de commande (s) pour l'interruption du traitement ophtalmologique si un mouvement de l'œil est détecté par le détecteur de mouvement (5), lequel se situe au-dessus d'une valeur de seuil de tolérance définie.

16. Dispositif de traitement ophtalmologique (30) comprenant un système laser (300) pour le traitement ophtalmologique de l'œil d'un patient (E), une tête d'application (3), et une interface de patient (2) pour appliquer la tête d'application (3) sur l'œil du patient (E), le dispositif de traitement ophtalmologique (30) comprenant, pour la fixation de l'interface ophtalmologique de patient (2) sur l'œil du patient (E), un dispositif de dépression (1) selon l'une des revendications 1 à 10.
